Europäisches Patentamt

(19) European Patent Office

Office européen des brevets

(11) Publication number: **0 453 190 A2**

(12) **EUROPEAN PATENT APPLICATION**

(21) Application number: **91303251.2**

(22) Date of filing: **12.04.91**

(51) Int. Cl.$^5$: **G01N 31/00**

(30) Priority: **17.04.90 JP 100802/90**

(43) Date of publication of application:
**23.10.91 Bulletin 91/43**

(84) Designated Contracting States:
**BE DE FR GB IT NL**

(71) Applicant: **OSAKA SANSO KOGYO LIMITED**
**Sumitomo Seimei Shin-Osaka Kita Building**
**1-14 Miyahara 4-Chome**
**Yodogawa-Ku Osaka 532 (JP)**

(72) Inventor: **Nakayasu, Toshihiro**
**5-5 Koshien Goban-cho**
**Nishinomiya City Hyogo Prefecture (JP)**
Inventor: **Nakahara, Yoshiyuki**
**2-19-16 Senbon-kita Nishinari-ku**
**Osaka (JP)**
Inventor: **Suzu, Yasuto**
**2-1812-21 Wakaba-cho Oumihachiman City**
**Shiga Prefecture (JP)**

(74) Representative: **Wickham, Michael**
**c/o Patent and Trademark Department The**
**BOC Group plc Chertsey Road**
**Windlesham Surrey GU20 6HJ (GB)**

(54) Method of analysing for carbon monoxide in a gas.

(57) A method of measuring CO concentration in a gas mixture containing at least some nitrogen comprises converting the CO in a sample to $CO_2$ using a metal oxide and measuring the resulting increase in $CO_2$ concentration of the sample by mass spectrometry. particularly atmospheric pressure ionisation mass spectrometry.

EP 0 453 190 A2

The present invention relates to the measurement of carbon monoxide in a gas containing at least some nitrogen.

There has been a rapid increase in the number of high-technology fields that require a high-precision analytical technique for controlling a trace component in a gas. Examples of such fields are the manufacture of semiconductors and superconductors. Such manufacturing processes often require steps to be performed in, for example, atmospheres consisting of or based on nitrogen. Even traces of impurity such as carbon monoxide can have a substantial, deleterious effect on the quality of the finished product.

There is therefore a need for ever more sensitive methods of analysing for carbon monoxide. Thus, recently, the analysis of carbon monoxide in a gas has been required to be able to detect the gas not only when present at the parts per million (ppm) level but also at the parts per billion (ppb) level.

It might first be considered to use mass spectrometry to analyse for carbon monoxide. However, when nitrogen is present, difficulties arise when analysing for CO in such a gas by such a method since both gases have the same mass number, i.e. 28.

Another possible method of analysing for CO in such a gas is by gas chromatography/mass spectrometry, which is performed by connecting together a gas chromatograph and a mass spectrometer. In this case, when the gas is passing through the gas chromatograph, the peak of $N_2$ and that of CO are somewhat offset from each other. In the case, for example, of a gas containing a trace amount of $N_2$ and a trace amount of CO, it is therefore possible to analyse CO in the gas by this method. However, in the case of a gas containing a large amount of $N_2$ and a trace amount of CO, it is impossible to determine the CO concentration exactly because the nitrogen peak becomes excessively large. Improvements can be obtained by focusing the mass spectrometer on the C-12 atoms present in the carbon monoxide, but the sensitivity of such a method is limited to several tens of parts per billion.

Recently when using an atmospheric pressure ionisation mass spectrometer, a technique of analysing for carbon monoxide by krypton addition has been developed. The limit of the detection is about 15 ppb.

We have conducted extensive studies in order that the detection sensitivity in the analysis of CO in a gas containing at least $N_2$ is able to be improved to the order of 1 ppb. (By 1 ppb is meant 1 part by volume per billion). As a result, we have found that, if a trace amount of CO in a gas under analysis is brought into contact with a metallic oxide, under heating if necessary, then CO is substantially completely converted into $CO_2$. The present invention uses this finding.

According to the present invention there is provided a method of measuring the concentration of carbon monoxide in an inert gas containing nitrogen gas or in nitrogen gas by use of mass spectrometry, (if desired, together with an initial gas chromatography step) comprising converting carbon monoxide in a gas under analysis into carbon dioxide preferably by use of a metal oxide, and analysing the resulting carbon dioxide by mass spectrometry.

The invention also provides apparatus for measuring trace impurities of carbon monoxide in nitrogen or a mixture of an inert gas and nitrogen, comprising a reactor adapted to convert the carbon monoxide to carbon dioxide and a mass spectrometer having an inlet communicating with an outlet of the reactor.

The metal oxide is preferably a copper oxide, for example CuO.

As has been described above, a trace amount of CO in a gas can be 100% converted to $CO_2$ by use of a metallic oxide, Since the mass number of $CO_2$ is 44, even a trace amount of $CO_2$ can be analysed by mass spectrometry. The sensitivity of detection of $CO_2$ is also extremely high.

A method according to the present invention will now be described by way of example and with reference to the accompanying drawings, in which:

Fig. 1 is a block diagram of an apparatus for carrying out one embodiment of the present invention.

Fig. 2 shows a calibration curve of carbon monoxide that is obtained by the apparatus shown in Fig. 1.

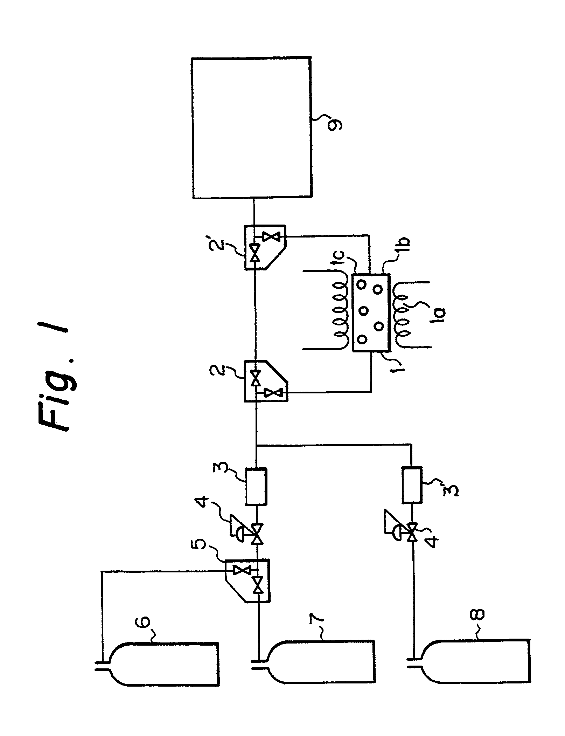

Referring to Fig. 1, there is shown a reaction furnace 1, which comprises a container 1b that is heated by a heater 1a and a metallic oxide 1c that is accommodated in the container 1b. One end of the reaction furnace 1 is connected through a switching valve 2 to three gas cylinders, that is, a purified nitrogen gas cylinder 6, a sample gas cylinder 7 and a carbon monoxide standard gas cylinder 8. The other end of the reaction furnace 1 is connected to an atmospheric pressure ionisation mass spectrometer 7 through a switching valve 2'.

The purified nitrogen gas 6 is introduced at a predetermined rate to the mass spectrometer 9 through a switching valve 5, a pressure reducing valve 4, a mass flow controller 3, the switching valve 2, the reaction furnace 1 and the switching valve 2'.

Next, the carbon monoxide standard gas 8 is introduced at a predetermined rate to the mass spectrometer 9 through a pressure reducing valve 4', a mass flow controller 3', the switching valve 2, the reaction furnace 1 and the switching valve 2'. The relative ionic strength of carbon dioxide at this time is measured. A calibration curve of carbon monoxide is prepared by varying the proportions of the purified nitrogen gas and the carbon monoxide standard gas.

Next, the sample gas 7 is introduced at a predetermined rate to the mass spectrometer 9 through the switching valve 5, the pressure reducing valve 4, the mass flow controller 3, the switching valve 2 and the switching valve 2', thereby by-passing the furnace 1'. The relative ionic strength (a) of carbon dioxide at this time is measured. By this operation, the content of carbon dioxide in the (unreacted) sample gas is measured.

Next, the sample gas from the cylinder 7 is introduced to the mass spectrometer 9 through the switching valve 5, the pressure reducing valve 4, the mass flow controller 3, the switching valve 2, the reaction furnace 1 (the carbon monoxide present all being converted we believe, by reaction with CuO to carbon dioxide, the CuO being reduced to Cu metal) and the switching valve 2'.

Accordingly, the carbon monoxide content of the gas is converted to carbon dioxide in the furnace 1.

The relative ionic strength of carbon dioxide (b) at this time is measured and is therefore representative of the total concentration of carbon monoxide and carbon dioxide in the unreacted sample gas. From the thus obtained relative ionic strength (b) of carbon dioxide in the sample gas, the previously determined ionic strength (a) of carbon dioxide contained in the sample gas is subtracted, and the resulting relative ionic strength (b-a) which is representative of the concentration of carbon monoxide is substituted into a suitable calibration equation. Thus, it is possible to measure the concentration of carbon monoxide in the sample gas.

The CuO is preferably in the finely divided form of a catalyst. Suitable copper oxide catalysts are commercially available from Englehard Inc. The CuO requires regeneration after use. This is preferably done by reoxidation at elevated temperature. Thus, the used CuO is reacted with oxygen. The temperature is carefully controlled so as to prevent sintering of the particles. If desired, the reoxidation may be performed in steps using nitrogen-oxygen mixtures of progressively greater oxygen mole fraction. For example, a 95 mole % nitrogen, 5 mole % oxygen mixture may be used in the first step, a 90 mole % nitrogen-10 mole % oxygen mixture in the second step, and so on. The temperature of the catalyst in the first step is desirably kept no higher than 250°C. A temperature sensor (e.g. a thermocouple) may be located in the CuO bed to enable the temperature to be monitored.

## Example 1

Cuprous oxide which had previously been heated in nitrogen gas at 350°C was charged into a reaction furnace 1.

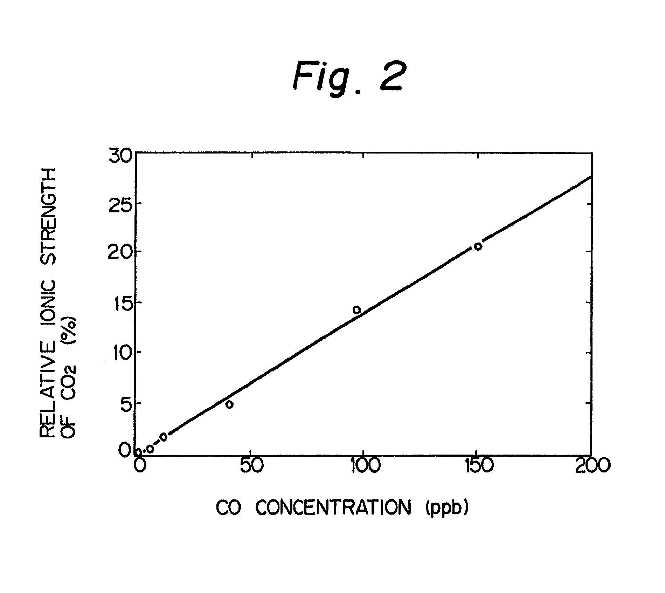

Purified nitrogen gas from the cylinder 6 was fed to atmospheric pressure ionisation mass-spectrometer 9 through the reaction furnace 1. Then, a definite amount of carbon monoxide standard gas from the cylinder 8 was fed through the reaction furnace 1 to the atmospheric pressure ionisation mass spectrometer 9. A calibration curve of carbon monoxide (1-150 ppb) was obtained as shown in Fig. 2 by changing the ratio of nitrogen gas to carbon monoxide.

Thereafter, a sample gas from the cylinder 7 was fed to the atmospheric pressure ionisation mass spectrometer 9 without passing through reaction furnace 1, whereby the relative ionic strength of $CO_2$ (a%) was measured. The $CO_2$ content in the unreacted sample gas was measured by this process. Then, the sample gas from the cylinder 7 was fed to the atmospheric pressure ionisation mass spectrometer through the reaction furnace 1 and the relative ionic strength of $CO_2$ (b%) in the reacted sample gas was measured. The part of the relative ionic strength of the reacted gas sample attributable to its original carbon monoxide content was then calculated by the formula:

$$b\% - a\% = c\%$$

and the actual carbon monoxide concentration was read off from the calibration curve shown in Figure 2.

## Example 2

A very small amount of CO in nitrogen gas was measured by the method of Example 1. The $CO_2$ content and CO content in so called 'ultra high purity' nitrogen which is used in the semiconductor industry were found to be 1.3 ppb and 4.8 ppb, respectively.

## Control Run 1

The CO content in the same gas as the gas employed in Example 2 was measured by a krypton addition method. The analysis wrongly indicated that the CO content was 22.5 ppb.

## Claims

1. A method of measuring the concentration of carbon monoxide in an inert gas containing nitrogen gas or in nitrogen gas using mass spectrometry, comprising converting carbon monoxide in a gas under analysis into carbon dioxide and analysing the resulting carbon dioxide by mass spectrometry.

2. A method according to Claim 1, in which the carbon monoxide is converted to carbon dioxide by reaction with oxygen in the presence of a metal oxide.

3. A method according to Claim 2, wherein the metal oxide is CuO or another oxide of copper.

4. A method according to any one of the preceding claims, in which the resulting carbon dioxide is analysed by atmospheric pressure ionisation mass spectrometry.

5. A method according to any one of the preceding claims, additionally including the steps of separately analysing the gas for carbon dioxide by mass spectrometry without converting its carbon monoxide content to carbon dioxide, and thereby obtaining a measurement representative of the carbon dioxide content of the unreacted gas, whereby the carbon dioxide measurement obtained for the reacted gas is able to be corrected by subtracting therefrom the measurement for the unreacted gas so as to obtain a valve representative of the carbon monoxide concentration.

6. Apparatus for measuring trace impurities of carbon monoxide in nitrogen or a mixture of an inert gas and nitrogen, comprising a reactor adapted to convert the carbon monoxide to carbon dioxide and a mass spectrometer having an inlet communicating with an outlet of the reactor.

7. Apparatus according to claim 6, additionally including valve means whereby the reactor is able to be by-passed.

8. Apparatus according to claim 6 or claim 7, in which the reactor includes a metal oxide.

9. Apparatus according to claim 8, in which the metal oxide is an oxide of copper.

10. Apparatus according to any one of claims 6 to 8, in which the mass spectrometer is of the atmospheric pressure ionisation kind.

# Fig. 1

Fig. 2